Europäisches Patentamt

European Patent Office

Office européen des brevets

(11) Numéro de publication: **0 336 047 B1**

# FASCICULE DE BREVET EUROPEEN

(45) Date de publication de fascicule du brevet: **12.08.92**   (51) Int. Cl.5: **A61L  2/20**

(21) Numéro de dépôt: **88400830.1**

(22) Date de dépôt: **06.04.88**

(54) **Appareil et procédé de stérilisation.**

(43) Date de publication de la demande:
**11.10.89 Bulletin  89/41**

(45) Mention de la délivrance du brevet:
**12.08.92 Bulletin  92/33**

(84) Etats contractants désignés:
**BE CH DE ES FR GB IT LI NL**

(56) Documents cités:
EP-A- 0 109 352
EP-A- 0 153 995

**Documentation technique Societé
"CIE.AEROVAP" 19. Rue de la Varenne - F. 94
Saint-Maur "Sterivap GL Automatique"**

(73) Titulaire: **Mercey, Gilles
14, Route d'Azé
F-41100 Vendome(FR)**

(72) Inventeur: **Mercey, Gilles
14, Route d'Azé
F-41100 Vendome(FR)**

(74) Mandataire: **Mongrédien, André et al
c/o BREVATOME 25, rue de Ponthieu
F-75008 Paris(FR)**

Rank Xerox (UK) Business Services

## Description

La présente invention a pour objet un appareil de stérilisation utilisable notamment en milieu hospitalier et fonctionnant par création de vapeurs bactéricides qui sont ensuite véhiculées dans des conduits jusqu'à une enceinte dans laquelle se trouve le corps à stériliser ; elle a également pour objet un procédé de stérilisation utilisable avec cet appareil.

D'après un appareil déjà connu de ce type, on verse du formol dans un récipient dont la paroi latérale est en verre et qui possède un fond métallique auquel est adaptée une résistance chauffante. Un courant de gaz porteur tel que l'air comprimé souffle sur la surface du formol chauffé et prélève donc les vapeurs qui sont émises. L'air mêlé aux vapeurs pénètre ensuite dans une enceinte dans laquelle se trouve le produit à stériliser ; quand la stérilisation a été menée à son terme, l'atmosphère de l'enceinte est renouvelée à l'aide d'un courant d'air pur.

Cet appareil présente l'inconvénient d'employer du formol, qui est susceptible de cristalliser et qui nécessite donc une inspection visuelle du bol chauffant, ce qui explique pourquoi la paroi de ce bol est en verre, mais ce qui représente cependant une construction plus compliquée et plus coûteuse.

Le même appareil peut être utilisé en employant comme fluide générateur des vapeurs bactéricides un mélange d'acide peracétique et d'eau ; des réactions chimiques assez compliquées se produisent au cours desquelles l'acide peracétique se transforme en acide acétique et libère des vapeurs riches en oxygène et donc bactéricides. Le chauffage favorise ici aussi la génération de ces vapeurs. Mais on ne peut empêcher la création de vapeur d'eau que le courant d'air entraîne, qui se condense ensuite dans le tuyau de l'installation et nécessite de prévoir des condenseurs entre le bol chauffant et l'enceinte à stériliser.

D'autres appareils tels que celui décrit dans le brevet européen EP-A1-0 109 352 sont caractérisés par l'introduction d'un bol de liquide dans l'enceinte à stériliser. L'évaporation de ce liquide produit les vapeurs bactéricides mais impose d'instaurer le vide dans l'enceinte et éventuellement de chauffer celle-ci, ce qui nécessite une pompe à vide et n'est pas forcément applicable à tous le corps à stériliser. L'enceinte à vide doit par ailleurs présenter une étanchéité suffisante.

La présente invention permet de remédier à ces inconvénients. Elle concerne un stérilisateur fonctionnant par extraction de vapeurs bactéricides engendrées par hydrolyse de l'acide peracétique, dans lequel l'extraction des vapeurs ainsi créées est effectuée non par soufflement d'un courant d'air au-dessus de la surface liquide mais par aspiration de ces vapeurs et incorporation au gaz porteur.

Plus spécifiquement, l'invention concerne un appareil de stérilisation par circulation de vapeurs bactéricides comprenant un générateur de courant de gaz porteur, un réservoir dans lequel les vapeurs bactéricides et de la vapeur d'eau sont produites par évaporation d'un liquide bactéricide en solution, un circuit de canalisations reliant le générateur de courant de gaz porteur à l'enceinte, caractérisé en ce que le circuit comprend un organe par lequel s'écoule le courant de gaz porteur qui aspire les vapeurs bactéricides du réservoir avant de les mêler au gaz porteur.

De façon avantageuse, les vapeurs sont créées par hydrolyse de l'acide peracétique et chauffage du mélange ; le réservoir est alors entièrement métallique et d'un seul bloc et présente une faible hauteur par rapport à ses dimensions horizontales. Il peut avantageusement être constitué d'un tube long et couché.

L'organe peut être constitué par un rétrécissement du circuit de canalisation créant une dépression dans le gaz porteur et une canalisation de raccordement entre le rétrécissement et une zone du réservoir emplie de vapeurs bactéricides.

L'invention concerne également un procédé de stérilisation par circulation de vapeurs bactéricides vers une enceinte de stérilisation, caractérisé en ce que les vapeurs bactéricides ainsi que de la vapeur d'eau sont produites par évaporation d'un liquide bactéricide en solution, puis aspirées hors d'un réservoir où le liquide séjourne et incorporées à un courant de gaz porteur.

Le mode de réalisation préféré de l'invention va à présent être décrit de façon plus concrète à l'aide des figures données en annexe à titre illustratif et non limitatif, dans lesquelles :

- la figure 1 représente un stérilisateur suivant l'art antérieur ;
- la figure 2 représente un stérilisateur objet de l'invention.

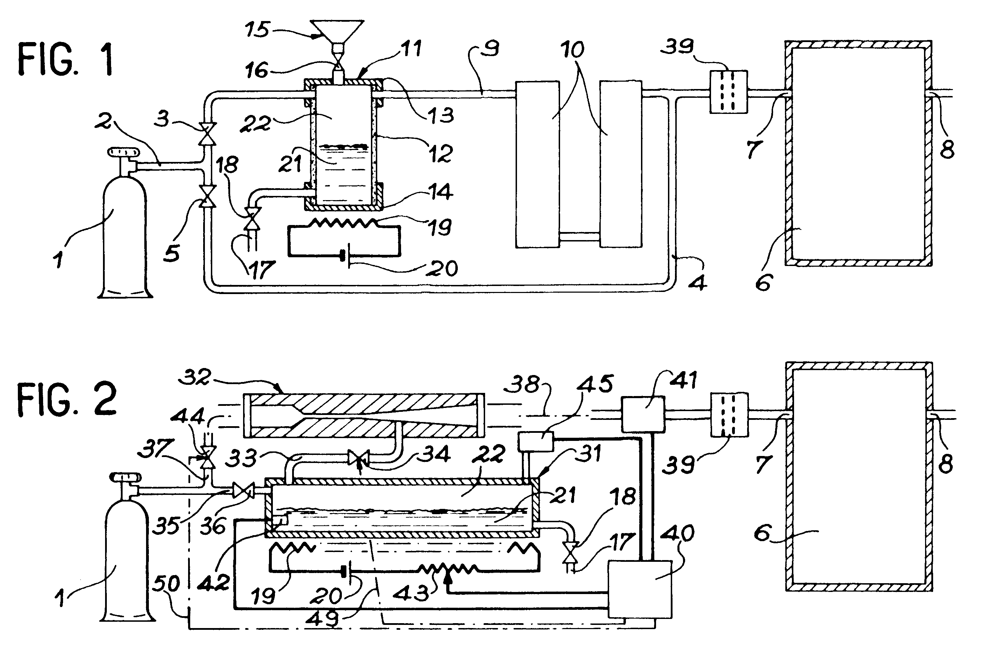

D'après l'appareil de la figure 1 connu et déjà commercialisé, la stérilisation se fait par circulation d'un courant d'air comprimé - constituant un gaz porteur - émis à partir d'une bouteille 1 vers une enceinte 6 dans laquelle se trouve le produit à stériliser. L'air passe tout d'abord par un tuyau 2 muni d'un moyen d'obstruction tel qu'une électrovanne 3 qui est ouverte au moment de la stérilisation ; l'air passe ensuite à travers un bol chauffant 11 puis dans un tuyau 9, une paire de condenseurs 10 et un orifice d'arrivée 7 dans l'enceinte 6. Un orifice d'évacuation 8 est prévu dans l'enceinte 6 pour éviter toute surpression.

Le bol chauffant 11 se décompose principalement en trois parties : une partie centrale 12 qui est en fait un paroi latérale transparente, et deux extrémités supérieure 13 et inférieure 14 en métal. La partie supérieure 13 sert essentiellement au raccordement du dispositif de remplissage 15 et des tuyaux 2 et 9 ; la partie inférieure 14 sert au raccordement du dispositif de vidange 17 et se trouve en outre en contact avec une résistance chauffante 19 alimentée par une source de courant électrique 20.

Au cours de la stérilisation, le bol chauffant 11 est rempli partiellement d'un liquide 21 susceptible d'engendrer, notamment par chauffage, des vapeurs bactéricides qui composent au moins partiellement l'atmosphère 22 à l'intérieur du bol chauffant 11.

La fabrication d'un bol chauffant 11 partiellement transparent est nécessaire quand le liquide 21 est du formol : ce corps est en effet susceptible de cristalliser et finalement de boucher les orifices d'entrée et de sortie du bol chauffant 11, ce qui nécessite de suivre visuellement l'évolution du phénomène.

Cette construction n'est pas nécessaire quand le liquide 21 est un mélange d'acide peracétique et d'eau ; par contre, les vapeurs produites par l'évaporation de ce mélange et que l'on retrouve dans l'atmosphère 22 du bol chauffant 11 sont un mélange des vapeurs bactéricides qui seules sont utiles et de vapeur d'eau. La production de vapeur d'eau est d'ailleurs favorisée par le soufflement de l'air comprimé sur la surface libre du liquide 21. Cet air, qui était très sec quand il était contenu dans la bouteille 1 (à une pression de 6 bars, l'air à 20°C peut contenir 3g d'eau par litre), se charge fortement d'humidité au cours de son transit dans le bol chauffant 11 (il peut contenir 50g de vapeur d'eau par litre à pression ambiante et à une température de 40°C régnant dans le bol chauffant 11), ce qui peut poser de sérieux problèmes de condensation dans la mesure où le débit d'air est usuellement de l'ordre de 40l par minute. C'est pourquoi il est nécessaire de disposer le long du tuyau 9 des condenseurs 10, ici au nombre de deux, qui retiennent la vapeur d'eau avant que le refroidissement de l'air jusqu'à la température ambiante ne force celle-ci à se condenser dans les tuyaux ou dans le filtre 39, ou dans l'enceinte 6. Ces condenseurs 10 présentent néanmoins l'inconvénient d'être encombrants et de compliquer l'installation.

Quand la stérilisation s'est poursuivie pendant un temps suffisant, on effectue un rinçage de l'atmosphère de l'enceinte 6 par une circulation d'air comprimé. A cet effet, on ferme l'électrovanne 3 et on ouvre l'électrovanne 5, ce qui permet à l'air comprimé de passer par un tuyau 4 reliant directement la bouteille d'air 1 à l'enceinte 6.

D'après cette conception, le remplacement du liquide 21 usé s'effectue par gravité : la vidange par ouverture d'un robinet 18 du dispositif de vidange 17, et le remplissage du bol chauffant 11 par du liquide neuf par ouverture d'un robinet 16 dans le dispositif de remplissage 15.

Si l'on se réfère à présent à la figure 2 qui détaille l'invention, on constate que l'air comprimé s'écoule à partir de la bouteille 1 et d'un tuyau 37 (l'électrovanne 44 étant ouverte) dans une trompe à vide 32 constituée principalement d'un venturi convergent-divergent, puis dans un tuyau 38 en communication avec l'enceinte 6 dans laquelle se trouve le produit à stériliser. L'équivalent du bol chauffant est ici un tube 31 long et couché dans lequel se trouve, comme précédemment, le mélange liquide 21 qui produit les vapeurs bactéricides et qui est surmonté d'une atmosphère 22. Ce tube 31 communique avec trois tuyaux : un tuyau d'aspiration 33, un tuyau de refoulement 35 et un tuyau de vidange 17. Le tuyau d'aspiration 33 communique avec la trompe à vide 32 et débouche dans une section resserrée du venturi, dans laquelle l'air comprimé a été fortement détendu et se trouve porté à une basse pression. Il en résulte que l'atmosphère 22 contenue dans le tube 31 est aspirée par le tuyau d'aspiration 33 et se mélange à l'air dans le trompe à vide 32. L'aspiration à l'aide d'une trompe à vide 32 permet une extraction beaucoup plus efficace des vapeurs bactéricides que par simple circulation d'air dans le bol chauffant 11 comme d'après l'art antérieur.

Un autre avantage important de ce procédé consiste en ce que l'air provenant de la bouteille 1 reste à la température ambiante, ce qui fait qu'il se charge moins volontiers en vapeur d'eau, qui est ainsi amenée à se condenser dans le tuyau d'aspiration 33 puis à retomber dans le tube 31. Pour cette raison, la trompe à vide 32 surmonte avantageusement le tube 31. Les condenseurs 10 deviennent donc inutiles.

Plusieurs détails de conception peuvent encore être apportés pour améliorer le fonctionnement et l'utilisation de ce stérilisateur. Il est tout d'abord possible, dans le cas où le liquide 21 est un mélange d'eau et d'acide peracétique, de renoncer à un bol chauffant 11 constitué de trois parties et d'utiliser un tube 31 uniquement en acier, donc de conception beaucoup plus simple. Le choix d'un choix d'un tube couché s'explique par l'avantage d'un réservoir présentant une surface de séparation entre le liquide 21 et l'atmosphère 22 qui soit grande par rapport à son volume, ce qui facilite l'extraction des vapeurs du liquide 21. Il est clair que, de ce point de vue, d'autres formes de réservoirs telles que des parallélépipèdes plats pourraient également convenir ; mais un tube permet en outre une vidange de liquide 21 usé et un remplissage de liquide 21 neuf plus efficace, comme on va l'expliquer plus loin.

Le stérilisateur d'après l'invention peut comprendre avantageusement un régulateur 40 qui, en fonction des données recueillies par un analyseur d'oxygène 41 disposé le tuyau 38 et d'un thermostat 42 qui mesure la température dans le tube 31, de modifier l'intensité du chauffage du liquide 21 en agissant sur un dispositif tel qu'une résistance variable 43 disposée en série avec la résistance chauffante 19.

Un dispositif de mesure de pression 45 est également prévu et agit sur l'électrovanne 34 à l'aide d'un dispositif d'asservissement connu 49 pour maintenir la dépression adéquate dans l'enceinte 31.

L'atmosphère 22 est alors portée à une pression de 0,8 à 0,9 bar, ce qui favorise l'extraction des vapeurs.

L'aspiration à une pression inférieure risquerait d'abaisser trop fortement ce qu'on appelle le point éclair de l'atmosphère 22, c'est-à-dire la température au-dessus de laquelle cette atmosphère 22 devient inflammable.

En outre, le dispositif de mesure de pression 45 peut également agir sur l'électrovanne 44 à l'aide d'un dispositif d'asservissement connu 50, ce qui limite la consommation en air comprimé et accroît fortement la concentration en vapeurs bactéricides dans le tayau 38 et au-delà, améliorant encore le fonctionnement de l'appareil.

Un filtre à air 39 peut encore être prévu entre la bouteille 1 et l'enceinte 6 de manière à empêcher la pollution de celle-ci par les impuretés continues éventuellement dans l'air comprimé.

Le rinçage de l'atmosphère de l'enceinte 6 après que la stérilisation a été effectuée se fait simplement par fermeture du tuyau aspirateur 33 par une électrovanne 34 ; les vapeurs ne sont alors plus aspirées et c'est uniquement de l'air pur qui pénètre dans l'enceinte 6.

L'invention permet également de procéder un peu différemment de l'art antérieur pour le remplissage et la vidange du liquide 21.

Quand l'acide peracétique a cédé son oxygène et se trouve transformé en acide acétique, on procède à son remplacement tout d'abord en interrompant le chauffage, en fermant l'électrovanne 34 et en ouvrant le robinet 18 du tuyau de vidange 17. On ouvre ensuite une électrovanne 36 sur le tuyau 35 qui relie la source d'air comprimé au tube 31 : il se forme un courant d'air qui contribue à chasser plus efficacement le liquide 21.

Quand le tube 31 est vide, on amène un récipient, non représenté ici, contenant le mélange d'acide peracétique et d'eau de remplacement à la sortie du tuyau de tuyau de vidange 17, on ferme l'électrovanne 36 et on rouvre l'électrovanne 34. Le liquide de remplacement est alors aspiré dans le tube 31.

On voit que dans cet appareil, le dispositif de remplissage 15 de l'art antérieur est supprimé. Bien entendu, le procédé que l'on vient de décrire est beaucoup plus efficace dans le cas où le réservoir contenant le liquide est un tube 31 : on peut alors disposer le tuyau de vidange 17 à une extrémité de ce tube et les tuyaux 33 et 35 d'aspiration et de refoulement à l'autre extrémité, ce qui favorise l'écoulement du liquide.

On constate donc que le dispositif de stérilisation objet de l'invention fonctionne de façon plus efficace en assurant une meilleure extraction des vapeurs riches en oxygène et en captant infiniment moins de vapeur d'eau. Le remplissage du tube 31 par aspiration permet de se passer du dispositif de remplissage du bol chauffant et incite à remplacer ce bol par un petit réservoir tubulaire dont la conception est beaucoup plus simple. Au total, l'appareil objet de l'invention doit être sensiblement moins encombrant et moins coûteux que les précédents, et il montre que l'extraction des vapeurs bactéricides peut être effectuée sans devoir utiliser une pompe à vide.

Bien entendu, l'emploi d'une trompe à vide 32 comportant un venturi n'est pas obligatoire et tout autre moyen permettant d'assurer l'aspiration des vapeurs bactéricides et l'incorporation de celles-ci à un courant d'air doit être considéré comme équivalent.

**Revendications**

1.    Appareil de stérilisation par circulation de vapeurs bactéricides vers une enceinte de stérilisation (6), comprenant un générateur de courant de gaz porteur (2), un réservoir (31) dans lequel les vapeurs bactéricides et de la vapeur d'eau sont produites par évaporation d'un liquide bactéricide en solution (21), un circuit de canalisation reliant le générateur de courant de gaz porteur à l'enceinte, caractérisé en ce que le circuit comprend un organe (32, 33) par lequel s'écoule le courant de gaz porteur qui aspire les vapeurs bactéricides du réservoir (31) avant de les mêler au gaz porteur.

2.    Appareil de stérilisation selon la revendication 1, caractérisé en ce que l'organe comprend un rétrécissement (32) du circuit de canalisation créant une dépression dans le gaz porteur et une canalisation (33) de raccordement entre le rétrécissement et une zone du réservoir (31) emplie de vapeurs bactéricides.

3.    Appareil de stérilisation selon l'une quelconque des revendications 1 ou 2, dans lequel les vapeurs bactéricides sont produites par hydrolyse de l'acide peracétique et chauffage de ce mélange, caractérisé en ce que le réservoir (31) est métallique et présente une dimension verticale faible par rapport à son extension horizontale.

**4.** Appareil de stérilisation selon la revendication 3, caractérisé en ce que le réservoir (31) est un tube long et couché.

**5.** Appareil de stérilisation selon l'une quelconque des revendications 1 à 4, caractérisé en ce que le remplissage du réservoir (31) s'effectue par aspiration dans un conduit (17) utilisé également pour la vidange du réservoir.

**6.** Procédé de stérilisation par circulation de vapeurs bactéricides vers une enceinte de stérilisation (6), caractérisé en ce que les vapeurs bactéricides, ainsi que de la vapeur d'eau, sont produites par évaporation d'un liquide bactéricide en solution (21), puis aspirées hors d'un réservoir (31) où le liquide (21) séjourne et incorporées à un courant de gaz porteur.

## Claims

**1.** Sterilization apparatus by the circulation of bactericidal vapours to a sterilization enclosure (6), comprising a carrier gas flow generator (2), a tank (31) in which the bactericidal vapours and water vapour are produced by the evaporation of a dissolved bactericidal liquid (21) and a pipe system connecting the carrier gas flow generator to the enclosure, characterized in that the pipe system comprises a member (32, 33) through which flows the carrier gas stream and which sucks the bactericidal vapours from the tank (31) before mixing them with the carrier gas.

**2.** Sterilization apparatus according to claim 1, characterized in that the member incorporates a constriction (32) of the pipe system creating a vacuum in the carrier gas and a connecting pipe (33) between the constriction and an area of the tank (31) filled with bactericidal vapours.

**3.** Sterilization apparatus according to either of the clams 1 and 2, in which the bactericidal vapours are produced by hydrolysis of peracetic acid and the heating of said mixture, characterized in that the tank (31) is made from metal and has a small vertical size compared with its horizontal extension.

**4.** Sterilization apparatus according to claim 3, characterized in that the tank (31) is a long, horizontal tube.

**5.** Sterilization apparatus according to any one of the claims 1 to 4, characterized in that the tank (31) is filled by suction in a pipe (17) also used for draining the tank.

**6.** Sterilization process by the circulation of bactericidal vapours to a sterilization enclosure (6), characterized in that the bactericidal vapours and the water vapour are produced by the evaporation of a dissolved bactericidal liquid (21) and are then sucked out of a tank (31), where the liquid (21) remains, and are incorporated into a carrier gas flow.

## Patentansprüche

**1.** Sterilisiervorrichtung durch Zirkulation von bakteriziden Dämpfen in einer Sterilisationshülle (6) mit einem Generator für ein Trägergas (2), einem Reservoir (31), in dem die bakteriziden Dämpfe und der Wasserdampf durch Verdampfen einer in Lösung befindlichen bakteriziden Flüssigkeit (21) hergestellt werden, und einem Kanalisationskreislauf, der den Generator für ein Trägergas mit der Hülle verbindet, dadurch gekennzeichnet, daß der Kreislauf ein Element (32, 33) umfaßt, durch das der Trägergasstrom strömt, der die bakteriziden Dämpfe des Reservoirs (31) aufnimmt, bevor sie mit dem Trägergas vermischt werden.

**2.** Sterilisationsvorrichtung nach Anspruch 1, dadurch gekennzeichnet, daß das Element eine Verengung (32) für den Kanalisationskreislauf, die einen Unterdruck in dem Trägergas erzeugt, und eine Verbindungskanalisation (33) zwischen der Verengung und einer mit bakteriziden Dämpfen gefüllten Zone des Reservoirs (31) aufweist.

**3.** Sterilisationsvorrichtung nach einem der Ansprüche 1 oder 2, in der die bakteriziden Dämpfe durch Hydrolyse der Peressigsäure und Erwärmen dieser Mischung hergestellt werden, dadurch gekennzeichnet, daß das Reservoir (31) metallisch ist und eine geringe vertikale Dimension bezüglich seiner horizontalen Ausdehnung aufweist.

**4.** Sterilisationsvorrichtung nach Anspruch 3, dadurch gekennzeichnet, daß das Reservoir (31) ein langes, geschichtetes Rohr ist.

**5.** Sterilisationsvorrichtung nach einem der Ansprüche 1 bis 4, dadurch gekennzeichnet, daß das Füllen des Reservoirs (31) durch Ansaugen über eine Leitung (17), die ebenfalls für das Leeren des Reservoirs verwendet wird, stattfindet.

6. Sterilisationsverfahren durch Zirkulation von bakteriziden Dämpfen in einer Sterilisationshülle (6), dadurch gekennzeichnet, daß die bakteriziden Dämpfe ebenso wie der Wasserdampf durch Verdampfung einer bakteriziden in Lösung befindlichen Flüssigkeit (21) erzeugt werden, anschließend aus einem Reservoir (31), in dem sich die Flüssigkeit (21) befindet, angesogen werden und in den Trägergasstrom eingebracht werden.

FIG. 1

FIG. 2

EP 0 336 047 B1